# EUROPEAN PATENT SPECIFICATION

(11) **EP 4 306 104 B1**
(45) Date of publication and mention of the grant of the patent: **18.03.2026**
(21) Application number: 22791968.5
(22) Date of filing: 18.04.2022
(51) Int. Cl.: A61K 9/127, A61K 47/24, A61K 47/32, A61K 31/7036, A61P 31/12, A61P 31/14, C12Q 1/18, G01N 33/566

(54) **PHARMACEUTICAL COMPOSITION FOR PREVENTING OR TREATING VIRUS INFECTIONS, COMPRISING POLYMER NANODISCS**
PHARMAZEUTISCHE ZUSAMMENSETZUNG ZUR PRÄVENTION ODER BEHANDLUNG VON VIRUSINFEKTIONEN MIT POLYMERNANOSCHEIBEN
COMPOSITION PHARMACEUTIQUE POUR LA PRÉVENTION OU LE TRAITEMENT D'INFECTIONS VIRALES, COMPRENANT DES NANODISQUES POLYMÈRES

(30) Priority: 20.04.2021 KR 20210051407
(43) Date of publication of application: 17.01.2024
(73) Proprietor: Mvrix Co., Ltd., Gyeonggi-do 18469 (KR)
(72) Inventor: KWEON, Dae Hyuk, Seoul 04129 (KR); KIM, Mi Soo, Suwon-si Gyeonggi-do 16362 (KR); JUNG, Young Hun, Atlanta, Georgia, 30329 (US); MOON, Jeong Hui, Suwon-si Gyeonggi-do 16362 (KR); KIM, Kyeong Won, Suwon-si Gyeonggi-do 16360 (KR); YOON, Jeong Hyeon, Suwon-si Gyeonggi-do 16360 (KR); HWANG, Jae Hyeon, Suwon-si Gyeonggi-do 16360 (KR); MOON, Seok Oh, Gwacheon-si Gyeonggi-do 13835 (KR); KIM, Soo Min, Anyang-si Gyeonggi-do 13922 (KR); PARK, Young Seo, Suwon-si Gyeonggi-do 16362 (KR); KIM, Na Yeon, Yongin-si Gyeonggi-do 16940 (KR)
(74) Representative: RGTH
(86) International application number: PCT/KR2022/005512
(87) International publication number: WO 2022/225268

(56) References cited:
- KR-B1- 102 181 991
- US-A1- 2019 154 698
- US-A1- 2019 154 698
- TANAKA MASAFUMI ET AL: "Effects of charged lipids on the physicochemical and biological properties of lipid-styrene maleic acid copolymer discoidal particles", BIOCHIMICA ET BIOPHYSICA ACTA, ELSEVIER, AMSTERDAM, NL, vol. 1862, no. 5, 28 January 2020 (2020-01-28), XP086064123, ISSN: 0005-2736, [retrieved on 20200128], DOI: 10.1016/J.BBAMEM.2020.183209
- CHEN ANGELA, MAJDINASAB ELLEANA J., FIORI MARIANA C., LIANG HONGJUN, ALTENBERG GUILLERMO A.: "Polymer-Encased Nanodiscs and Polymer Nanodiscs: New Platforms for Membrane Protein Research and Applications", FRONTIERS IN BIOENGINEERING AND BIOTECHNOLOGY, vol. 8, XP055965513, DOI: 10.3389/fbioe.2020.598450
- P. BHATTACHARYA, S. GRIMME, B. GANESH, A. GOPISETTY, J. R. SHENG, O. MARTINEZ, S. JAYARAMA, M. ARTINGER, M. MERIGGIOLI, B. S. PRAB: "Nanodisc-Incorporated Hemagglutinin Provides Protective Immunity against Influenza Virus Infection", JOURNAL OF VIROLOGY, THE AMERICAN SOCIETY FOR MICROBIOLOGY, US, vol. 84, no. 1, 1 January 2010 (2010-01-01), US , pages 361 - 371, XP055289313, ISSN: 0022-538X, DOI: 10.1128/JVI.01355-09

## Description

### [Technical Field]

The present invention relates to a polymer nanodisc containing a lipid bilayer and an amphiphilic polymer, and a pharmaceutical composition containing the polymer nanodisc.

### [Background Art]

Influenza virus is an RNA virus that belongs to the Orthomyxoviridae family and is divided into three serotypes: influenza A, influenza B, and influenza C. Thereamong, influenza B and C viruses have been confirmed to infect only humans, while influenza A virus has been confirmed to infect humans, horses, pigs, other mammals, and various types of poultry and wild birds. The serotypes of influenza A virus are classified depending on the types of two proteins, namely, hemagglutinin (HA) and neuraminidase (NA), on the surface of the virus. There are 144 types (16 types of HA proteins and 9 types of NA proteins) that have been known to date. HA functions to attach the virus to somatic cells, and NA functions to allow the virus to penetrate into cells.

Drugs for viral infections that have been developed to date include M2 ion channel inhibitors such as amantadine and rimantadine, and neuraminidase inhibitors such as oseltamivir (Tamiflu^{®}) or zanamivir (Relenza^{®}), but these drugs have a drawback of limited effectiveness. In other words, it is known that mutant viruses resistant to amantadine or rimantadine-based derivative compounds are quickly formed, the H5N1 type influenza viruses detected in some regions are resistant to amantadine or rimantadine-based compounds, and influenza B virus is insensitive to amantadine-based derivatives. In addition, it is known that the number of resistant viruses against oseltamivir or zanamivir-based derivative compounds is increasing and such resistant viruses frequently occur in children.

Research is actively underway to develop novel drugs capable of overcoming the problems of conventional viral infection therapies as described above. For example, Korean Patent No. 1,334,143 discloses a composition for preventing or treating cold, avian influenza, swine influenza, or swine flu containing a Polygala karensium extract and a xanthone-based compound isolated therefrom. However, these substances are ineffective in preventing or treating influenza-derived diseases such as swine flu due to low anti-viral activity thereof.

In addition, many epidemics of diseases caused by viruses have occurred in recent years, such as MERS caused by a coronavirus in 2015 and COVID-19 caused by a coronavirus that has been ongoing since 2020.

Coronavirus is an RNA virus that belongs to the Coronavirinae subfamily of the Coronaviridae family and causes respiratory and digestive system infections in humans and animals. Coronavirus is mainly contracted through mucous membrane infection and droplet transmission, and generally causes mild respiratory infections in humans, but may also cause fatal infections, and may also cause diarrhea in cattle and pigs, and respiratory disease in chickens. Coronavirus is a representative virus that causes fatal infectious diseases in modern civilization. In April 2003, severe acute respiratory syndrome, also known as SARS, originated in the People's Republic of China spread, and many people died, with a mortality rate of 9.6%. In 2015, Middle East Respiratory Syndrome, also known as MERS, spread from the Middle East to the world, resulting in many deaths with a mortality rate of about 36%. In addition, as the new coronaviral infection (COVID-19) originating from Wuhan, China has been confirmed around the world since December 2019, the number of infected people has been increasing. The fatality rate is relatively low at 2.6% according to data collected through February 2020, but the number of confirmed cases is rapidly increasing around the world, and there are no vaccines or antiviral drugs approved for prevention or treatment therefor. Currently, drugs used to treat other diseases, such as camostat, remdesivir, and hydroxychloroquine, are being reviewed for the potential thereof as coronavirus drugs, and clinical trials have failed or halted due to side effects and minimal efficacy thereof.

Vesicular stomatitis Indiana virus (VSV), called "Indiana vesiculovirus", and "formerly Vesicular stomatitis", belongs to the Rhabdoviridae family. It is mainly a livestock infectious disease and there is no drug therefor.

All of the viruses described above have something in common: they have an outer envelope composed of a lipid bilayer. Many animal viruses including the viruses mentioned above (e.g., HIV, hepatitis virus, MERS, herpes virus, and Ebola virus) have membrane proteins and lipid bilayer envelopes. Membrane proteins or surface antigens exist on the outer shell of the virus and have the property of binding to receptors on host cells, leading the virus to penetrate into cells. In addition, the envelope not only plays a very important role in the viral infection path, but also protects itself from the human immune system. Therefore, as described above, viral infection can be inhibited by interfering with the binding between the membrane protein of the virus and the receptor or by damaging the envelope. Among drugs (virustatic drugs) that inhibit viral proliferation by interfering with the membrane protein of the virus, oseltamivir is known as a representative drug. However, such an antiviral drug has a temporary effect, allows for recurrence of viral infection, or has poor efficacy. This is because the drug acts to inhibit the proliferation of the virus and thus the virus proliferates again when the concentration of the drug is low. In addition, when proliferation and cessation are repeated upon changes in drug concentration, the virus may naturally become resistant, as a result of "adaptive evolution." Resistant viruses to oseltamivir (Tamiflu) are also increasingly being reported. It is not easy to develop drugs using neutralizing antibodies because simply inhibiting the viral infection cycle is not sufficient. Drugs that reduce the infectivity of enveloped viruses are called "virucides" and soaps and disinfectants are used as representative virucides in everyday life. However, virucides are not safe and thus use thereof as drugs is limited.

Therefore, the mechanism of action of virucides that damage the viral envelope is advantageous in order to obtain strong medicinal efficacy. In addition, there is a need for virucides that are safe and are applicable to a wide range of enveloped viruses. For this purpose, based on the fact that the upper region of the membrane protein common to viruses is positively charged, binding to the virus is induced using the negative charge of the structure and then a nanodisc structure that is capable of incorporating into and damaging the virus envelope is formed.

Drugs that destroy the outer shells of viruses are advantageously commonly applicable to mutant viruses because the mutation occurs in the viral protein, whereas the outer shell of the virus is derived from the cell membrane and is therefore not affected by the mutation of the virus. In addition, drugs that destroy the outer shell of viruses may have much stronger efficacy than conventional antiviral drugs. Therefore, the present invention aims at developing a powerful and safe antiviral agent applicable to a wide range of enveloped viruses by developing a virucide that non-selectively destroys the envelope of the virus after binding to the virus through electrostatic attraction.

### [Disclosure]

### [Technical Problem]

The present invention is designed to improve the stability of the structure and antiviral activity by replacing a membrane scaffold protein (MSP) with an amphipathic polymer in consideration of the fact that the membrane scaffold protein (MSP) in the conventional nanoperforator structure has low stability to heat and low resistance to proteases in the body. In addition, in order to form nanodiscs with MSP, complicated processes, such as mixing proteins with lipids using a surfactant such as sodium cholate and then removing the surfactant using an adsorbent such as biobead SM2 should be performed, whereas polymer nanodiscs are advantageously formed automatically by simply mixing with lipids. Further, polymer nanodiscs have a strong negative charge on the sides thereof, whereas nanodiscs produced from MSP do not have a strong negative charge thereon. The polymer nanodisc, which has a strong negative charge, interacts with the positive charge of the virus surface protein and thus is easily accessible to the virus, and easily inactivate the virus due to the activity of the nanodisc. This could provide a starting point for the development of antiviral drugs that are active against a wide range of viruses, and has the potential to dramatically increase the antiviral activity of nanodiscs. In other words, when MSP is replaced with an amphiphilic polymer in the nanodisc, it has advantages of having virucidal activity against a wide range of viruses, and higher antiviral performance than conventional nanodiscs, and of providing an easy preparation process. In addition, the use of polymer rather than proteins can provide much higher production efficiency.

As such, the present inventors have made extensive research efforts to develop novel antiviral agents that can replace conventional viral inhibitors. As a result, the present inventors found that the polymer-based nanodisc containing the lipid bilayer and the amphiphilic polymer exhibits excellent antiviral effects. Based thereon, the present invention has been completed.

Accordingly, it is one object of the present invention to provide a polymer-based nanodisc for use in preventing or treating viral infections containing a lipid bilayer and an amphiphilic polymer.

It is another object of the present invention to provide a pharmaceutical composition for use in preventing or treating viral infections containing the polymer-based nanodisc.

It is another object of the present invention to provide an optimized structure to improve the efficacy of the polymer-based nanodisc.

### [Technical Solution]

In accordance with an aspect of the present invention, the above and other objects can be accomplished by the provision of a polymer nanodisc for use in preventing or treating a viral infection having a configuration in which an amphipathic polymer is bound to a lipid bilayer including hydrophobic moieties of amphiphilic lipids contacting each other.

In an embodiment, preferably, the polymer nanodisc may have a configuration in which the hydrophobic moieties of amphiphilic lipids contacting each other are arranged as a bilayer including upper and lower layers to form a disc-shaped lipid bilayer, and parts of the hydrophobic moieties of the amphiphilic lipids exposed from side surfaces of the amphiphilic lipids are surrounded by an amphipathic polymer through a hydrophobic bond.

In an embodiment, preferably, the amphiphilic lipid may include at least one phospholipid selected from the group consisting of phosphatidylcholine, phosphatidylglycerol, phosphatidylethanolamine, and phosphatidylserine.

In an embodiment, preferably, the amphiphilic lipid may include at least one selected from the group consisting of DOPC (1,2-dioleoyl-sn-glycero-3-phosphocholine), DLPC (1,2-dilauroyl-sn-glycero-3-phosphocholine), DMPC (1,2-dimyristoyl-sn-glycero-3-phosphocholine), DPPC (1,2-dipalmitoyl-sn-glycero-3-phosphocholine), POPC (1-palmitoyl-2-oleoyl-sn-glycero-3-phosphocholine), C13PC, DDPC (1,2-didecanoyl-sn-glycero-3-phosphocholine), DSPC (1,2-distearoyl-sn-glycero-3-phosphocholine), DEPC (1,2-dierucoyl-sn-glycero-3-phosphocholine), DLOPC (1,2-dilinoleoyl-sn-glycero-3-phosphocholine), EPC (egg phosphatidylcholine), MSPC (1-myristoyl-2-stearoyl-sn-glycero-3-phosphocholine), PMPC (1-palmitoyl-2-myristoyl-sn-glycero-3-phosphocholine), PSPC (1-palmitoyl-2-stearoyl-sn-glycero-3-phosphocholine), SMPC (1-stearoyl-2-myristoyl-sn-glycero-3-phosphocholine), SPPC (1-stearoyl-2-palmitoyl-sn-glycero-3-phosphocholine) and combinations thereof.

In an embodiment, preferably, the amphiphilic polymer may be present in an amount of 0.1 to 20% based on a total weight of the polymer nanodisc.

In an embodiment, preferably, the polymer nanodisc may perforate a lipid bilayer envelope of a virus.

In an embodiment, preferably, the virus may include at least one selected from the group consisting of the families Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae and Herpesviridae.

In an embodiment, preferably, the virus may include at least one selected from the group consisting of influenza virus, coronavirus, and vesicular stomatitis virus.

In accordance with another aspect of the present invention, provided is a pharmaceutical composition for use in preventing or treating a viral infection containing the polymer nanodisc.

In accordance with another aspect of the present invention, provided is a method for screening an optimized polymer nanodisc structure composition including reacting a polymer nanodisc having a configuration in which an amphipathic polymer is bound to a lipid bilayer including hydrophobic moieties of amphiphilic lipids contacting each other with a target virus (a), and determining whether or not the polymer nanodisc exhibits antiviral activity against the target virus (b).

### [Advantageous effects]

The aspects and advantages of the present invention are summarized as follows:
(a) The present invention provides a polymer nanodisc for use in preventing or treating a viral infection containing a lipid bilayer and an amphiphilic polymer.
(b) The present invention provides a pharmaceutical composition for use in preventing or treating viral infections containing the polymer nanodisc.
(c) The polymer nanodisc containing the lipid bilayer and the amphiphilic polymer exhibits excellent antiviral effects and thus is applicable to prevention or treatment of viral infections.
(d) The amphiphilic polymer for use in preventing or treating a viral infection is found to exhibit greatly improved antiviral activity compared to conventional ApoAl or MSP.
(e) The present invention provides a method for screening an optimized nanodisc structure composition including reacting a polymer nanodisc and determining whether or not the polymer nanodisc exhibits antiviral activity.

### [Description of Drawings]

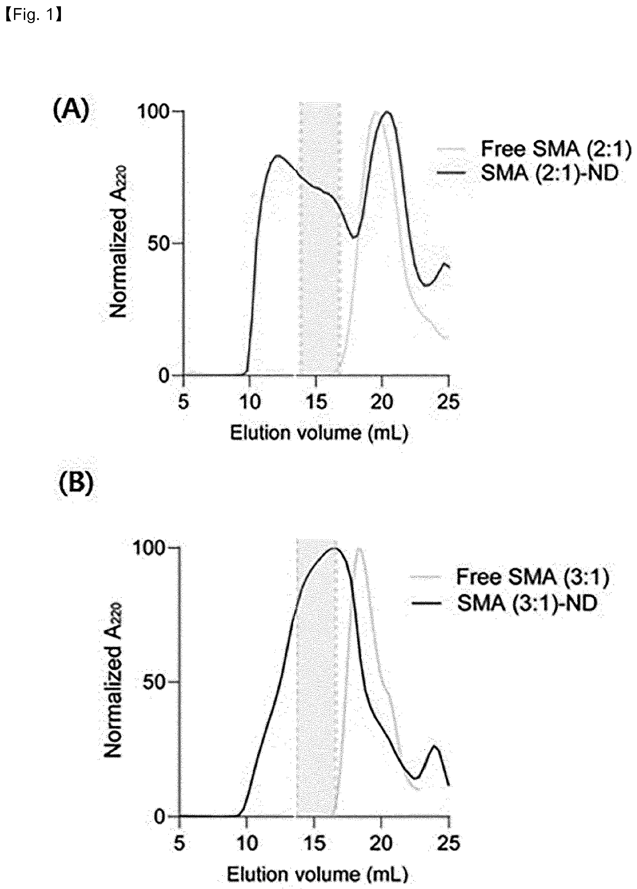

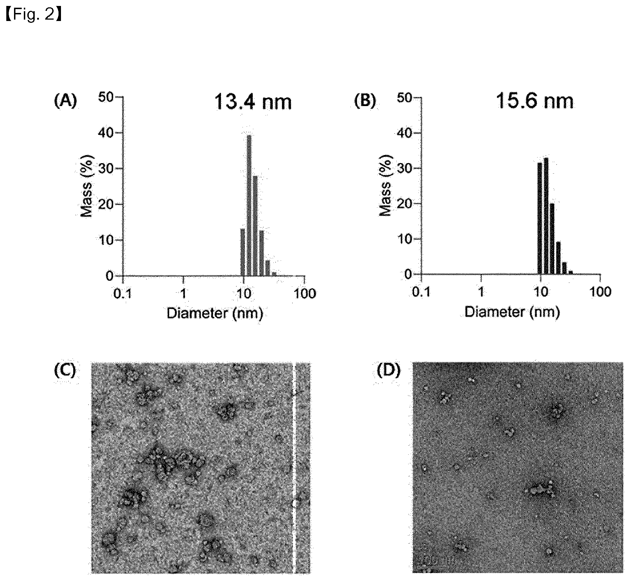

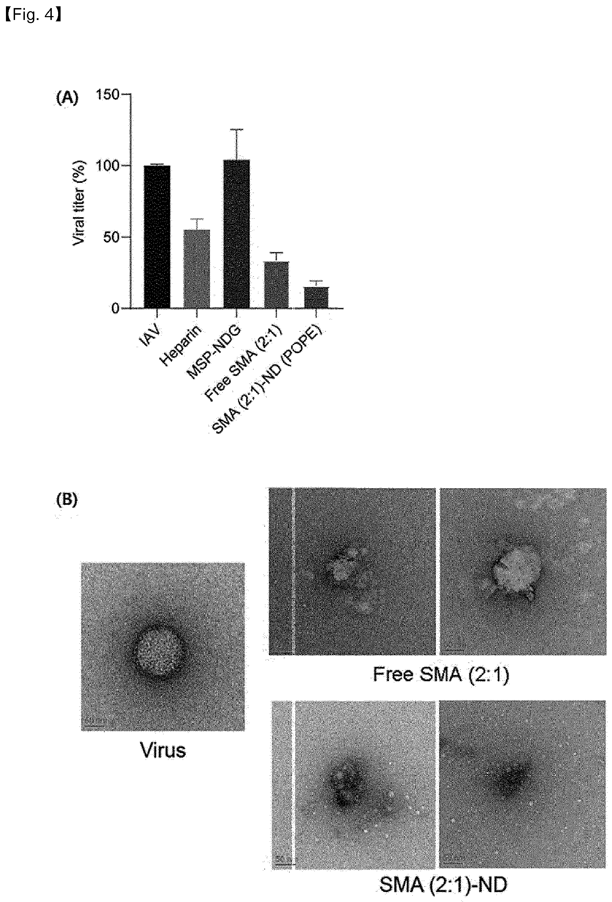

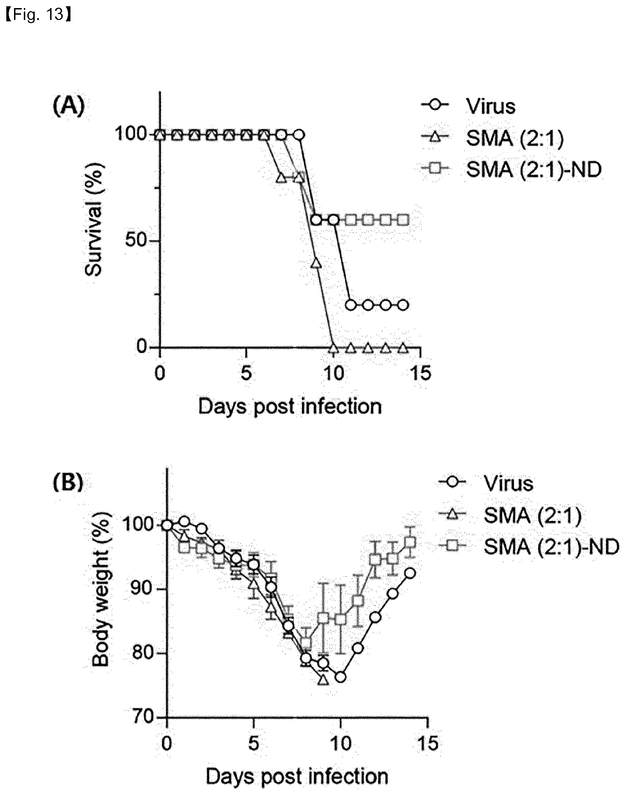

(A) of FIG. 1 shows the distribution pattern by size of the polymer (SMA 2:1) nanodisc (lipid:SMA = 1:2) purified through SEC, and (B) of FIG. 1 shows the distribution pattern by size of the polymer (SMA 3:1) nanodisc (lipid:SMA = 1:2) purified through SEC.
(A) of FIG. 2 is a graph showing the diameter distribution of the polymer (SMA 2:1) nanodisc (lipid:SMA = 1:2) measured through DLS, (B) of FIG. 2 is a graph showing the diameter distribution of the polymer (SMA 3:1) nanodisc (lipid: SMA = 1:2) measured through DLS, (C) of FIG. 2 is a transmission electron microscope image illustrating the structure of the polymer (SMA 2:1) nanodisc (lipid: SMA = 1:2), and (D) of FIG. 2 is a transmission electron microscope image illustrating the structure of the polymer (SMA 3:1) nanodisc (SMA 2:1).

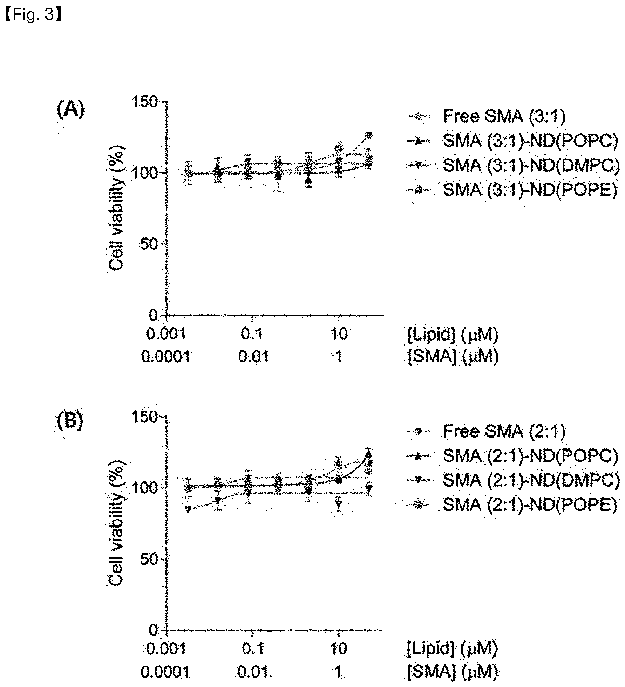

FIG. 3 shows the results of an experiment for testing the cytotoxicity of polymers (SMA 3:1 (A), SMA 2:1 (B)) and polymer nanodiscs in MDCK cells.
(A) of FIG. 4 shows the results of an experiment for testing the antiviral efficacy against influenza virus of polymer (SMA 2:1), polymer nanodisc, protein-based nanodisc (MSP-NDG), and heparin, and (B) of FIG. 4 is a transmission electron microscope image illustrating the shape changes of influenza virus due to the polymer (SMA 2:1) and polymer nanodisc.
FIG. 5 shows the results of an experiment for comparing the antiviral efficacy against influenza viruses of nanodiscs containing POPC, DPPC, DMPC, DOTAP, and POPE as phospholipids.
FIG. 6 shows the results of an experiment for testing an increase in thermal stability of the structure of the polymer nanodisc containing POPE phospholipid.
FIG. 7 shows the results of an experiment for comparing the antiviral efficacy of the polymer and polymer nanodisc against influenza in environments at different temperatures (4°C (top) and 37°C (bottom)).
FIG. 8 shows the results of an experiment for comparing the virucidal activity of the polymers, polymer nanodiscs (composed of POPC or POPE) and heparin drugs.
FIG. 9 shows the results of PCR (polymerase chain reaction) and Western blot to detect the presence of the reporter gene mCherry-luciferase viral RNA and spike protein in the coronavirus.
FIG. 10 shows the results of an experiment for confirming the antiviral efficacy of polymers and polymer nanodiscs against various mutant strains of influenza viruses.
FIG. 11 shows the results of an experiment for confirming the antiviral efficacy of polymers and polymer nanodiscs against VSV-pseudovirus.
FIG. 12 shows the results of an experiment for confirming the antiviral efficacy of polymers and polymer nanodiscs against coronavirus-pseudovirus.
(A) of FIG. 13 shows the results of an experiment for confirming the antiviral efficacy of the polymer nanodisc through an animal experiment (survival rate), and (B) of FIG. 13 shows the results of an experiment for confirming the antiviral efficacy of the polymer nanodisc through an animal experiment (weight change).

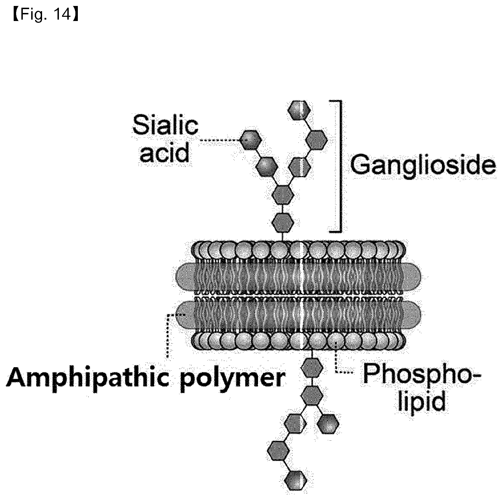

FIG. 14 is a schematic diagram illustrating a polymer nanodisc according to the present invention.

### [Best Mode]

In one aspect, the present invention is directed to a polymer nanodisc for use in preventing or treating a viral infection containing a lipid bilayer and an amphiphilic polymer.

As used herein, the term "nanodisc" refers to a unilamellar disc-shaped material containing a lipid bilayer and is characterized by having an open system in which both hydrophilic surfaces of the lipid bilayer are exposed to the outside. In other words, this may mean that the nanodisc itself according to the present invention does not form a closed space having an inner core through the lipid bilayer.

As used herein, the term "polymer" refers to a compound containing a molecule in which one or more structural units (monomers) are polymerized and linked to each other through a large number of chemical bonds. Depending on the structure of the polymer, the polymer is classified into a linear polymer, a branched polymer, and a networked polymer. A polymer including two or more different monomers is called a "copolymer" and a polymer including one monomer is referred to as "a homopolymer". In the present invention, among these polymers, an amphiphilic polymer is used.

Here, the term "polymer nanodisc" refers to a nanodisc further containing an amphipathic polymer in added to the nanodisc.

In one embodiment of the present invention, the polymer nanodisc for use in preventing or treating a viral infection preferably has a configuration in which hydrophobic moieties of amphiphilic lipids contacting each other are arranged as a bilayer, that is, upper and lower layers to form a disc-shaped lipid bilayer, and parts of the hydrophobic moieties of the amphiphilic lipids exposed from the side surfaces of the amphiphilic lipids are surrounded by an amphipathic polymer layer (amphipathic polymer moiety of FIG. 14) through a hydrophobic bond (FIG. 14).

The amphiphilic polymer for use in preventing or treating a viral infection is amphiphilic and thus serves to prevent the hydrophobic tail (acyl tail) from being directly exposed to water. When this amphipathic polymer is present in a predetermined rate, the polymer surrounds the intermediate layer of phospholipids (two-layer hydrophobic acyl tails) forming the lipid bilayer. As a result, the disc-shaped lipid bilayer is stabilized in an aqueous solution. At this time, the size of the lipid bilayer disc is determined depending on the ratio of the phospholipid to the polymer.

The lipid constituting the polymer nanodisc may be a phospholipid and may include a lipid tail having 1 to 50 carbon atoms, more specifically 5 to 30 carbon atoms.

In one embodiment of the present invention, the amphipathic lipid is preferably a phospholipid, and more preferably, the phospholipid may include at least one selected from the group consisting of phosphatidylcholine, phosphatidylglycerol, phosphatidylethanolamine, and phosphatidylserine and any lipid may be used without limitation so long as it is useful as a cell membrane ingredient.

The phosphatidylcholine may be DOPC (1,2-dioleoyl-sn-glycero-3-phosphocholine), DLPC 1,2-dilauroyl-sn-glycero-3-phosphocholine), DMPC (1,2-dimyristoyl-sn-glycero-3-phosphocholine), DPPC (1,2-dipalmitoyl-sn-glycero-3-phosphocholine), POPC (1-palmitoyl-2-oleoyl-sn-glycero-3-phosphocholine), Cl3PC, DDPC (1,2-didecanoyl-sn-glycero-3-phosphocholine), DSPC (1,2-distearoyl-sn-glycero-3-phosphocholine), DEPC (1,2-dierucoyl-sn-glycero-3-phosphocholine), DLOPC (1,2-dilinoleoyl-sn-glycero-3-phosphocholine), EPC (egg phosphatidylcholine), MSPC (1-myristoyl-2-stearoyl-sn-glycero-3-phosphocholine), PMPC(1-palmitoyl-2-myristoyl-sn-glycero-3-phosphocholine), PSPC (1-palmitoyl-2-stearoyl-sn-glycero-3-phosphocholine), SMPC (1-stearoyl-2-myristoyl-sn-glycero-3-phosphocholine) or SPPC (1-stearoyl-2-palmitoyl-sn-glycero-3-phosphocholine).

The phosphatidylglycerol may be DMPG (1,2-dimyristoyl-sn-glycero-3[phospho-rac-(1-glycerol)], DPPG (1,2-dipalmitoyl-sn-glycero-3[phospho-rac-(1-glycerol)]), DSPG (1,2-distearoyl-sn-glycero-3[Phospho-rac-(1-glycerol)), POPG (1-palmitoyl-2-oleoyl-sn-glycero-3[phospho-rac-(1-glycerol)]), DEPG (1,2-dierucoyl-sn-glycero-3[phospho-rac-(1-glycerol)]), DLPG (1,2-dilauroyl-sn-glycero-3[phospho-rac-(1-glycerol)]), DOPG (1,2-dioleoyl- glycero-3[phospho-rac-(1-glycerol)]) or DSPG (1,2-distearoyl-sn-glycero-3[phospho-rac-(1-glycerol)]).

The phosphatidylethanolamine may be DMPE (1,2-dimyristoyl-sn-glycero-3-phosphoethanolamine), DPPE (1,2-dipalmitoyl-sn-glycero-3-phosphoethanolamine), DSPE (1,2-distearoyl-sn-glycero-3-phosphoethanolamine), DOPE (1,2-dioleoyl-sn-glycero-3-phosphoethanolamine), DEPE (1,2-dierucoyl-sn-glycero-3-phosphoethanolamine), DLPE (1,2-dilauroyl-sn-glycero-3-phosphoethanolamine) or POPE (1-palmitoyl-2-oleoyl-sn-glycero-3-phosphoethanolamine).

The phosphatidylserine may be DOPS (1,2-dioleoyl-sn-glycero-3-phosphoserine), DLPS (1,2-dilauroyl-sn-glycero-3-phosphoserine), DMPS (1,2-dimyristoyl-sn-glycero-3-phosphoserine), DPPS (1,2-dipalmitoyl-sn-glycero-3-phosphoserine), DSPS (1,2-distearoyl-sn-glycero-3-phosphoserine) or POPS.

The amphipathic lipid constituting the polymer nanodisc, in addition to the phospholipid, may further contain at least one selected from the group consisting of neutral fats, such as triglycerides, cholesterol or derivatives thereof, and saccharolipids, such as ganglioside.

The polymer nanodisc that has a disc shape having an open system in which both hydrophilic surfaces of the lipid bilayer are exposed to the outside is distinguished from a spherical liposome that has a closed system in which a hydrophilic core is present inside and only one of the two surfaces of the lipid bilayer is exposed to the outside. The liposome can form a closed space including an inner core through the lipid bilayer.

In an embodiment of the present invention, the amphiphilic polymer for use in preventing or treating a viral infection may include at least one selected from the group consisting of styrene-maleic acid (SMA), di-isobutylene-maleic acid (DIBMA), styrene-maleimide (SMI), polymethyl methacrylate (PMA), and combinations thereof.

SMA, which is a copolymer in which styrene and maleic acid are polymerized in a ratio of 2:1 or 3:1, may be polymerized by a polymerization method widely known in the art. Further, SMA may be modified with amine, ethylenediamine, quaternary ammonium, sulfhydryl, or the like.

In one embodiment of the present invention, the amphiphilic polymer for use in preventing or treating a viral infection is preferably present in an amount of 0.1 to 20% based on the total weight of the polymer nanodisc. More specifically, the amphiphilic polymer is present in an amount of 0.2% to 5%, 0.3% to 5%, 0.4 to 5%, 0.1% to 4%, 0.2% to 4%, 0.3% to 4%, 0.4% to 4%, 0.1% to 3%, 0.2% to 3%, 0.3% to 3%, 0.4% to 3%, 0.1% to 2%, 0.2% to 2%, 0.3% to 2%, or 0.4% to 2%, based on the total weight of the polymer nanodisc.

According to an embodiment of the present invention, the amphiphilic polymer for use in preventing or treating a viral infection may be SMA and the SMA is preferably present in an amount of 0.3 to 0.7% of the total weight of the polymer nanodisc.

In one embodiment of the present invention, the polymer nanodisc for use in preventing or treating a viral infection may have a diameter of 2 to 50 nm, but is not limited thereto. More specifically, the polymer nanodisc has a diameter of 2 nm to 40 nm, 2 nm to 30 nm, 3 nm to 50 nm, 3 nm to 40 nm, 3 nm to 30 nm, 4 nm to 50 nm, 4 nm to 40 nm, 4 nm to 30 nm, 5 nm to 50 nm, 5 nm to 40 nm, or 5 nm to 30 nm, but is not limited thereto. According to an embodiment of the present invention, the type of polymer of the polymer nanodisc may be SMA and the diameter thereof may be 10 nm to 20 nm.

In one embodiment of the present invention, the polymer nanodisc for use in preventing or treating a viral infection was found to perforate the lipid bilayer envelope of a virus.

In an embodiment of the present invention, the virus may, for example, include at least one virus selected from the group consisting of the families *Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae* and *Herpesviridae.* More specifically, the virus includes: Sin Nombre hantavirus, etc., which belongs to the *Bunyaviridae* family; coronavirus, etc., involved in various acute respiratory syndromes, which belongs to the *Coronaviridae* family; Ebola virus, Marburg virus, etc., which belong to the *Filoviridae* family; West Nile virus, yellow fever virus, Dengue fever virus, Hepatitis C virus, etc., which belong to the *Flaviviridae* family; hepatitis B virus, etc., which belongs to the *Hepadnaviridae* family; herpes simplex 1 virus, herpes simplex 2 virus, etc., which belong to the *Herpesviridae* family; influenza virus etc., which belongs to the *Orthomyxoviridae* family; smallpox virus, vaccinia virus, molluscum contagiosum virus, monkeypox virus, etc., which belong to the *Poxviridae* family; Vesicular stomatitis virus, etc., which belongs to the *Rhabdoviridae* family; HIV (human immunodeficiency virus) etc., which belongs to the *Retroviridae* family; Chikungunya virus etc., which belongs to the *Togaviridae* family; and pseudorabies virus, HHV virus, etc., which belong to the *Herpesviridae* family.

In one embodiment of the present invention, the virus may include at least one selected from the group consisting of influenza virus, coronavirus, and vesicular stomatitis virus.

In another aspect, the present invention is directed to a pharmaceutical composition for use in preventing or treating a viral infection containing the polymer nanodisc.

The pharmaceutical composition for use in preventing or treating a viral infection of the present invention contains the polymer nanodisc for use in preventing or treating a viral infection, which is directed to the aspect of the present invention, and thus duplicate description is omitted to avoid excessive redundancy in the specification.

As used herein, the term "viral infection" refers to a disease caused by infection with a virus having a lipid bilayer envelope. Examples of the viral infection include: hemorrhagic fever with renal syndrome (epidemic hemorrhagic fever) caused by infection with a virus of the family *Verniaviridae*; respiratory diseases such as coryza caused by infection with a virus of the family *Coronaviridae*; hepatitis C caused by infection with a virus of the family *Flaviviridae*; hepatitis B caused by infection with a virus of the family *Hepadnaviridae*; herpes zoster caused by infection with a virus of the family *Herpesviridae*; flu or influenza viral infection caused by infection with a virus of the family *Orthomyxoviridae*; smallpox caused by infection with a virus of the family *Poxviridae*; rabies or bullous stomatitis caused by infection with a virus of the family *Rhabdoviridae*; acquired immunodeficiency syndrome, etc. caused by infection with a virus of the family *Retroviridae*; and the like. As another example, the viral infection may be flu or influenza viral infection caused by infection with an influenza virus that belongs to the family *Orthomyxoviridae.*

The pharmaceutical composition of the present invention may contain a pharmaceutically acceptable carrier in addition to the composition as an active ingredient.

The pharmaceutically acceptable additive contained in the pharmaceutical composition of the present invention may be commonly used in the formulation and includes, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate and mineral oil.

In addition to the ingredients, the pharmaceutical composition of the present invention may further contain lubricants, wetting agents, sweeteners, flavoring agents, emulsifiers, suspending agents, preservatives, and the like. Suitable pharmaceutically acceptable carriers and agents are described in detail in Remington's Pharmaceutical Sciences (19th ed., 1995).

The pharmaceutical composition of the present invention may be administered orally or parenterally, for example, intrathecally, intravenously, subcutaneously, intradermally, intramuscularly, intraperitoneally, intrasternally, intratumorally, intranasally, intracerebrally, intracranially, intrapulmonarily, and intrarectally, but is not limited thereto.

That is, the pharmaceutically effective amount of the pharmaceutical composition of the present invention may vary depending on the formulation method, administration method, age, weight, gender, pathological condition, diet, administration time, administration route, excretion rate and reaction sensitivity of the patient. Ordinary skilled physicians can easily determine and prescribe an effective dosage (pharmaceutically effective amount) for desired treatment or prevention. According to a preferred embodiment of the present invention, the daily dose of the pharmaceutical composition of the present invention is about 0.0001 to about 100 mg/kg.

As used herein, the term "pharmaceutically effective amount" means an amount sufficient to prevent or treat the disease.

As used herein, the term "prevention" refers to any action that suppresses or delays the onset of a disease by administration of the pharmaceutical composition according to the present invention. As used herein, the term "treatment" refers to any action that reduces, inhibits, ameliorates or removes disease conditions.

The pharmaceutical composition of the present invention is formulated into a unit dose form or packaged into a multiple dose container using a pharmaceutically acceptable carrier and/or excipient in accordance with a method that can be easily performed by those skilled in the art. The formulation may be prepared in a variety of forms, such as an oral drug or injection, may be in the form of a solution, suspension or emulsion in an oil or aqueous medium, or in the form of an extract, a powder, a suppository, a powder, a granule, a tablet or a capsule, and may further contain a dispersant or stabilizer.

As used herein, the term "administration" or "administering" means directly administering a therapeutically or prophylactically effective amount of the composition of the present invention to a subject suffering from, or likely to suffer from, the disease of interest, so that the same amount accumulates in the body of the subject.

As used herein, the term "therapeutically effective amount" refers to the content of the composition sufficient to impart a therapeutic or prophylactic effect to the subject to whom the composition is administered, and is meant to include a "prophylactically effective amount."

In addition, as used herein, the term "subject" refers to mammals including humans, mice, rats, guinea pigs, dogs, cats, horses, cows, pigs, monkeys, chimpanzees, baboons, and rhesus monkeys. Most specifically, the subject of the present invention is a human.

In another aspect, the present invention is directed to a method for screening an optimized nanodisc structure composition including reacting a polymer nanodisc having a configuration in which an amphipathic polymer is bound to a lipid bilayer including hydrophobic moieties of amphiphilic lipids contacting each other with a target virus (a) and determining whether or not the polymer nanodisc exhibits antiviral activity against the target virus (b).

Meanwhile, in step (b), the method for determining whether or not the polymer nanodisc exhibits anti-viral activity against viruses is not particularly limited and the method may be a well-known method such as NA activity inhibition analysis, hemagglutination reaction inhibition analysis, RNA release analysis, plaque reduction analysis, or a combination thereof.

Meanwhile, through experiments, the present applicant has identified that the polymer nanodisc of the present invention i) does not exhibit cytotoxicity, ii) has excellent stability, and iii) has antiviral efficacy against various viruses such as influenza virus, coronavirus, and vesicular stomatitis virus. In addition, the present applicant identified an increase in antiviral efficacy by varying the types of phospholipids and polymers among the components of the structure.

Therefore, the polymer nanodisc of the present invention exhibits excellent stability, ease of production, excellent antiviral efficacy, and low cytotoxicity, and can perform excellent antiviral functions in vivo.

Hereinafter, the present invention will be described in more detail with reference to the following examples. It will be obvious to those skilled in the art that these examples are provided only for better understanding of the present invention and thus should not be construed as limiting the scope of the present invention based on the subject matter of the present invention.

Throughout this specification, "percentage (%)" used to indicate a concentration of a specific substance means (weight/weight) % for solid/solid, (weight/volume) % for solid/liquid, and (volume/volume)% for liquid/liquid, unless mentioned otherwise.

### [Example 1: Production of polymer nanodisc]

To produce polymer nanodiscs, two types of polymers prepared by polymerization from styrene and maleic acid in SMA (styrene-maleic acid) in a ratio of 2:1 and 3:1 were used as polymers and 16:0-18:1 POPC (phosphatidylcholine, abbreviated below), 16:0 DPPC (dipalmitoylphosphatidylcholine, abbreviated below), 14:0 DMPC (1,2-dimyristoyl-sn-glycero-3-phosphocholine, abbreviated below), 18:1 DOTAP (dioleoyl-3-trimethylammonium propane, hereinafter abbreviated), 16:0-18:1 POPE (1-palmitoyl-2-oleoyl-sn-glycero-3-phosphoethanolamine, hereinafter abbreviated) were used as constituent lipids. Each ingredient was used in this experiment is as follows.

**[Table 1]**

| Name | Ratio of lipid:SMA (w/w) |
|---|---|
| SMA (2:1)-ND (POPC) | 1:2 |
| SMA (2:1)-ND (DPPC) | 1:1 |
| SMA (2:1)-ND (DMPC) | 1:1 |
| SMA (2:1)-ND (POPE) | 1:4 |
| SMA (2:1)-ND (DOTAP) | 1:3 |
| SMA (3:1)-ND (POPC) | 1:2 |
| SMA (3:1)-ND (POPE) | 1:4 |

| | |
|---|---|
| * ND = the method of producing polymer nanodisc is specifically as follows. | |

First, lipids in the form of a solid powder were dissolved in an organic solvent and then the lipids of the composition in Table 1 above were mixed in a glass tube, and then the organic solvent was completely vaporized using nitrogen gas and a vacuum tube to allow only the lipids to be left in the form of a film. After the film-shaped lipids were sufficiently dissolved for more than 5 minutes using hydration buffer (10 mM HEPES, 150 mM NaCl; pH 7.4), frozen and thawed more than 5 times using liquid nitrogen and water at 50°C to produce liposomes. Then, the polymer was added in an amount of 0.5 to 2% (w/v), and frozen and sonicated more than 5 times at 50°C using liquid nitrogen and a sonicator. As a result, as the polymer penetrates the liposome, water molecules enter to form pores between the lipid layers, ultimately forming a nanodisc structure. Ultracentrifugal filtration was performed to remove polymers that did not form nanodiscs and liposomes with large diameters. The concentrate of the completed nanodisc was purified depending on size through size exclusion chromatography and the obtained sample was concentrated (FIG. 1 (A), (B)). The lipid concentration in the nanodisc was measured using a lipid quantification assay kit and the concentration of the sample was expressed based on the lipid concentration.

### [Example 2: Confirmation of structure and shape of polymer nanodisc]

### 1) Confirmation of structure of polymer nanodisc

The diameter of the polymer nanodisc produced in Example 1 was measured through DLS (dynamic light scattering). The result showed that, when 1.2% SMA (2:1) was added to 10 mM lipid (POPC 100%), the average diameter of the polymer nanodisc was 13.4 nm and the weight of the polymer nanodisc was 80.9% of the total weight ((A) in FIG. 2). In addition, when 1.2% SMA (3:1) was added to 10 mM lipid (POPC 100%), the average diameter of the polymer nanodisc was 15.6 nm and the weight of the polymer nanodisc was 87.4% of the total weight (FIG. 2(B)).

### 2) Confirmation of structure of polymer nanodisc

In order to observe the structure and shape of the polymer nanodisc, the diameter of which was confirmed, lipids and polymers were diluted to the total concentration of 20 nmol/100 µL and imaged using a transmission electron microscope (SMA 2:1 in FIG. 2(C), and SMA 3:1 in FIG. 2(D)). The formation of the disc structure using SMA according to the present invention is performed by producing a structure using a method similar to the present invention by combining phospholipids with SMA and imaging the shape of the structure with a transmission electron microscope, as described in "Tanaka M et al., Effects of charged lipids on the physicochemical and biological properties of lipid-styrene maleic acid copolymer discoidal particles. Biochim Biophys Acta Biomembr. (2020) 3-4."

### [Example 3: Verification of cytotoxicity of polymer nanodiscs]

When a polymer that is not synthesized in vivo is injected in the body as a drug, the polymer may be recognized as a foreign antigen, causing an immune response or toxicity. Whether or not two types of polymers and polymer nanodiscs exhibit cytotoxicity was determined in consideration of this.

2*10⁴ cells/100 µL of MDCK cells were seeded in a 96 well plate. After 24 hours, when the cell confluency reached over 90%, the cells were treated with 1/5 serial dilutions of polymers from the highest concentration of 5 µM to the lowest concentration of 0.00032 mM and 1/5 serial dilutions of polymer nanodiscs from the highest concentration of 50 µM to the lowest concentration of 0.0032 based on phospholipids. This is based on the consideration that a ratio of the polymer in the nanodisc with a diameter of 10 nm is 1/10. After 24 hours, when treated with a WST-8 solution, which is a soluble tetrazolium salt, at a density of 100 µL/well, the WST-8 is reduced by an enzyme called "dehydrogenase" in living cells, to form orange formazan. Using this mechanism, the percentage of living cells (cell viability) was measured. Both the polymer and polymer nanodisc did not exhibit cytotoxicity ((A) and (B) of FIG. 3).

### [Example 4: Verification of antiviral efficacy of polymer nanodisc]

### 1) Confirmation of antiviral efficacy of various drugs including polymer and polymer nanodisc

Microneutralization assay (MN) was performed to determine whether or not the polymer and polymer nanodisc actually have antiviral efficacy.

Nanodisc (MSP-nanodisc, MSP-NDG) produced using materials used in the experiment (heparin, membrane scaffold protein), Free-SMA (2:1), and SMA (2:1)-ND (POPE) were set at the identical concentration (10 µM based on phospholipid concentration, 1 µM based on polymer concentration) and influenza virus (A/Puerto Rico/8/34) was treated therewith at 37°C for 1 hour. MDCK cells, which had been seeded at 2*104 cells/100 µL in a 96-well plate the day before and had reached 100% confluency, were treated with the mixture of the materials and the virus that had been reacted for 1 hour. After 24 hours, the result was treated at a concentration of 0.5 µM with 4-MUNANA (4-methylumbelliferyl-N-acetyl-a-D-neuraminic acid) and reacted at 37°C for 1 hour. The fluorescence at 355/460 nm was measured to quantitatively analyze the NA (neuraminidase) activity of the virus.

It was found that, based on the same concentration, the antiviral efficacy of polymer free SMA (2:1) and polymer nanodisc SMA (2:1)-ND (POPE) was superior to the conventional drugs, heparin and MSP-NDG ((A) of FIG. 4). At this time, free SMA itself also has antiviral activity due to amphipathicity thereof, but tends to aggregate. However, free SMA produced in the form of a nanodisc is advantageously free from aggregation and adopts a variety of constituent phospholipids. In fact, the following experiment showed that when nanodiscs were produced with different phospholipid compositions and efficacies thereof were compared, the structure containing POPE phospholipids was the most effective. In addition, the fact that nanodisc is more effective than free SMA in vivo is considered to be due to the improved stability and efficacy of the structure.

### 2) Confirmation of changes in virus shape through transmission electron microscopy

To determine whether or not the shape of the virus was actually destroyed by the polymer and polymer nanodisc, 1 µM of the polymer and 10 µM of the polymer nanodisc, based on the phospholipid concentration, were reacted with the influenza virus (A/Puerto Rico/8/34) for 1 hour and imaged using a transmission electron microscope ((B) of FIG. 4). As a result, it was observed by the naked eye that the virus was destroyed to the extent that it could be distinguished from a normal virus with the naked eye.

### [Example 5: Confirmation of increased antiviral efficacy and increased stability through changes in phospholipid composition in polymer nanodisc]

### 1) Confirmation of increased antiviral efficacy through changes in phospholipid composition within polymer nanodisc

In order to detect the antiviral effect of nanodisc depending on the type of phospholipid, the antiviral efficacy of nanodiscs using various phospholipids was verified.

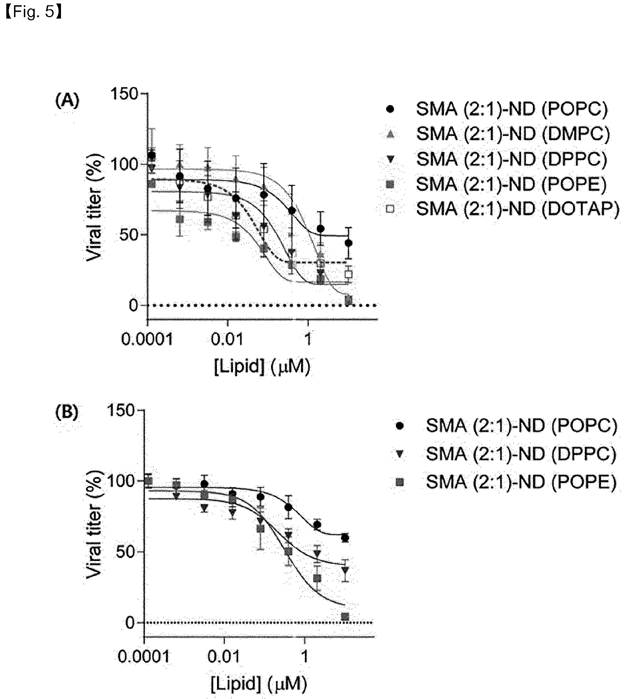

Polymer nanodiscs were produced using phospholipids other than POPC, such as DMPC, DPPC, POPE, and DOTAP, and then serially diluted in 1/5 from the highest concentration based on phospholipids, 10 µM, and then influenza virus (A/Puerto Rico/8/34) was treated with the resulting dilution at 37°C for 1 hour. The subsequent process was performed in the same manner as in Example except that the phospholipid composition was different. At this time, (A) and (B) of FIG. 5 show the results of experiments with five and three different types of phospholipids which were conducted to re-verify the improvement in efficacy and re-compare the phospholipids. The result of the experimentation showed that the polymer nanodisc composed of POPE among phospholipids exhibit improved antiviral efficacy compared to nanodiscs composed of other phospholipids, and had IC₅₀ of 0.21 µM.

### 2) Confirmation of increased stability (stability to temperature) through changes in phospholipid composition within polymer nanodiscs

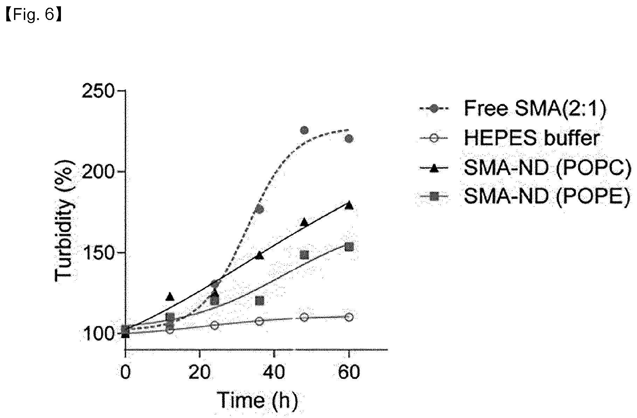

The stability of the polymer and polymer nanodiscs produced from POPC and POPE, respectively, as phospholipids, in a 10 mM HEPES-based aqueous solution, was determined by measuring the change in turbidity over time at 37°C (FIG. 6). The result showed that the stability against temperature was high when the polymer was converted into nanodisc, and among the phospholipids in the nanodisc, POPE was found to exhibit the highest stability.

### [Example 6: Difference in antiviral activity between polymer and polymer nanodisc]

### 1) Differences in antiviral activity between polymer and polymer nanodisc under temperature conditions

The difference in antiviral activity between the polymer and the polymer nanodisc at different temperatures (4°C and 37°C) was detected through MN assay. The polymer and polymer nanodisc used in the assay were serially diluted 1/5 from the highest concentration of 1 µM for the polymer and 10 µM for the phospholipids in the nanodisc, and incubated with influenza virus (A/Puerto Rico/8/34) at 4°C, 37°C for 1 hour. MDCK cells, which had been seeded in a 96-well plate at 2*10⁴ cells/100 µl the day before and had reached 100% confluency, were treated with the mixture of the materials and virus that had been reacted for 1 hour. After 1 hour, the plate was incubated again at 37°C for 1 hour, and then the drug and virus mixture were removed and the medium was replaced with a fresh medium. After 24 hours, the result was treated with 4-MUNANA (4-methylumbelliferyl-N-acetyl-a-D-neuraminic acid) at a concentration of 0.5 µM and reacted at 37°C for 1 hour. The fluorescence at 355/460 nm was measured to quantitatively analyze the NA (neuraminidase) activity of the virus.

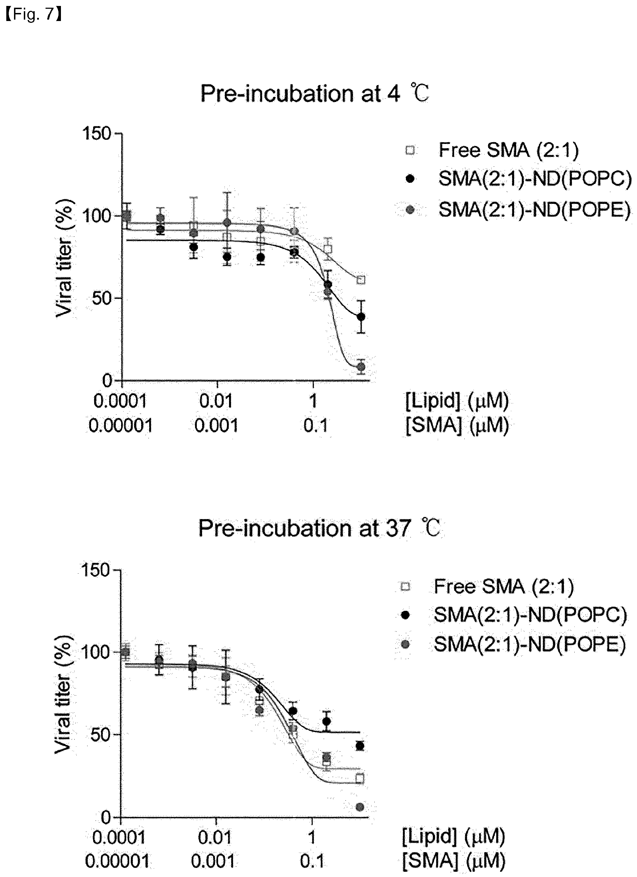

The result showed that the antiviral activity of the polymer decreased at 4°C, compared to at 37°C, and the polymer nanodisc containing POPE among phospholipids had greater antiviral activity than the polymer both at 4°C and 37°C (FIG. 7).

### 2) Differences in virucidal activity between polymer and polymer nanodisc

In order to detect the antiviral action mechanism of the polymer and polymer nanodisc, compared to membrane protein-based nanodisc and heparin, the virus was reacted with the drug at a concentration of EC₉₀ at 37°C for 1 hour and then diluted to a concentration of 1/100. The MDCK cells that had been seeded were treated with the resulting dilution. After 24 hours, the cells were treated with 4-MUNANA (4-methylumbelliferyl-N-acetyl-α-D-neuraminic acid) at a concentration of 0.5 µM and reacted at 37°C for 1 hour. The fluorescence at 355/460 nm was measured to quantitatively analyze the NA (neuraminidase) activity of the virus.

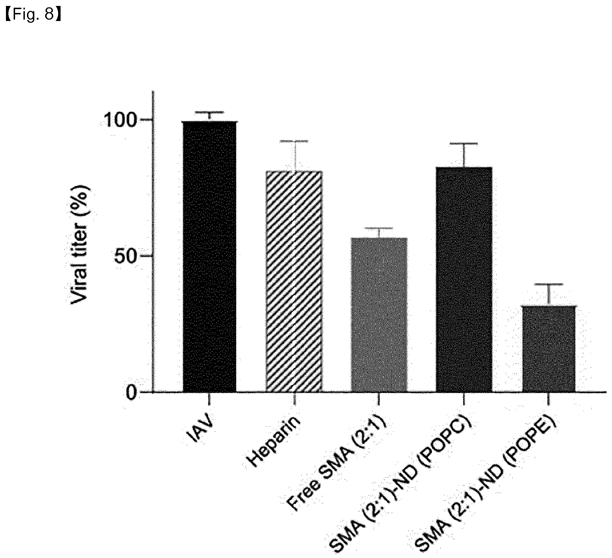

Heparin, a conventional drug known to be virustatic activity had a recovery in viral activity even when diluted at 1/100, whereas the polymer and polymer nanodisc had a low viral activity even when diluted at 1/100 (FIG. 8). This indicates that the mechanism of action of the drug is similar to that of virucide. In particular, the nanodisc containing phospholipid POPE was found to have the best virucidal activity.

### [Example 7: Production of VSV (vesicular stomatitis virus)-pseudovirus, coronavirus-pseudovirus]

### 1) Production of plasmids for production of dual reporter VSV-pseudovirus and coronavirus-pseudovirus

The HIV gag-pol plasmid (pNL4.3-mCherry-Luc) required for the production of two pseudoviruses was purchased from Addgene (Massachusetts, USA). Information on the plasmid is described in Kara G. Lassen, et al.. 2012, A Flexible Model of HIV-1 Latency Permitting Evaluation of Many Primary CD4 T-Cell Reservoirs, PLOS one. VSV-G and mVSV-G(H162R) plasmids were acquired from another laboratory (KIST, Korea) and related information was acquired from Cell Biolabs (California, USA). The coronavirus spike protein was purchased from Sino Biological (Beijing, China), and C-term 19 amino acid deletion was performed through site directed mutagenesis. The sequences of the primers used in the experiment are listed in Table 2 below. The detailed composition and reaction conditions are listed in Table 3.

**[Table 2]**

| | |
|---|---|
| Forward primer | TGTGGCTCCTGTTGTTAAAAGTTTGATGAGGAT (SEQ ID NO: 1) |
| Reverse primer | ATCCTCATCAAACTTTTAACAACAGGAGCCACA (SEQ ID NO: 2) |

**[Table 3]**

| | |
|---|---|
| PCR composition | Each primer 1 µL, vector 3 µL, dNTP 1 µL, 5X Phusion GC buffer 10 µL, Phusion polymerase 0.5 µL and distilled water 33.5 µL |
| PCR conditions | Initial denaturation: 98°C, 3 mins |
| | 30 cycles repetition: denaturation (98°C, 20 seconds) → annealing (61°C, 30 seconds) → extension (72°C, 6 minutes) |
| | Final extension: 72°C, 10 minutes |

### 2) Plasmid Maxiprep for transformation

Top10 soluble cells were transformed with the purchased and constructed plasmid using a heat shock method for maxiprep to perform transformation. Colonies grown on solid medium containing 150 µg/ml of ampicillin were inoculated into LB broth (Miller) containing 10 ml of ampicillin and incubated at 37°C in a 250 rpm shaking incubator for 16 hours. Then, the colonies were secondarily inoculated into 600 ml of the same liquid medium and incubated in a shaking incubator at 37°C and 200 rpm for an additional 16 hours. Then, the result was centrifuged at 6,000 rpm for 10 minutes to remove the medium and only the cells were obtained. The plasmid was purified in a large amount from the obtained cells using the Maxiprep kit (Qiagen, USA).

### 3) VSV-pseudovirus, coronavirus-pseudovirus production

Pseudovirus was produced from 293T cell line (ATCC, USA) and purified. The 293T cell line was subcultured in a culture medium prepared by adding 50 ml of FBS (ATLAS, USA) and 5 ml of PSN (Hyclone, USA) to 445 ml of DMEM (Hyclone, USA). 15 µg of HIV gag-pol plasmid, 5 µg of VSV-G plasmid, 60 µg of PEI and 15 µg of HIV gag-pol plasmid, 5 µg of spike protein plasmid, and 60 µg of PEI were added to the 293T cell line incubated at a density of about 90% in a 10 cm dish. 60 µg was added and cultured in an incubator at 37°C and 5% CO2 for 8 hours, then the medium was replaced with fresh one, and the result was incubated for an additional 48 hours. Then, the culture medium was collected and separated from the cells by centrifugation at 800 g for 5 minutes, and then 20% sucrose cushion centrifugation was performed thereon as needed.

### 4) Confirmation of characteristics of produced VSV-pseudovirus and coronavirus-pseudovirus

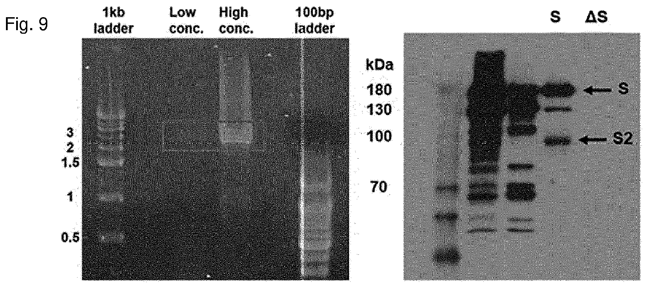

Whether or not mCherry-luciferase viral RNA and outer membrane protein (corona spike protein) were present in the two pseudoviruses was determined. The sequences of the primers used in the experiment are listed in Table 4 below. The pseudovirus product was treated with 1% Triton X-100, PCR (polymerase chain reaction) was performed using M-MLV reverse transcriptase (Cosmogenetech, Korea) and KOD-Neo-Plus (Toyobo, Japan), and electrophoresis on a 1 agarose gel was performed at 135V for 30 minutes to detect amplification. Detailed composition and reaction conditions for the corresponding PCR reaction are listed in Tables 5 and 6. The presence of the outer membrane protein was detected by Western blotting. First, the pseudovirus product was centrifuged at 23,000 rpm for 2 hours 30 minutes, concentrated, loaded on 8% PAGE-gel, and electroporated at 80V for 20 minutes and at 140V for 1 hour and 10 minutes. Then, PAGE-gel was placed on a nitrocellulose membrane (NC membrane) and a current of 270 mA was applied thereto for 40 minutes to transfer proteins onto the NC membrane. Then, 5% skim milk powder (w/v) was dissolved in TBST buffer and NC membrane blocking was performed for 1 hour. Anti-SARS-CoV-2 spike antibody (abcam, USA) was dissolved in TBST at a concentration of 1 µg/ml and the NC membrane was treated with the resulting antibody for 1 hour. Then, the result was washed with TBST for 10 minutes and this process was repeated 3 times. Anti-rabbit IgG antibody (Sigma, USA) was dissolved in TBST buffer at a ratio of 1:2,000, the result was treated with the antibody for 1 hour and then washed again with TBST for 10 minutes. This process was repeated three times. Then, a chemiluminescence reaction was induced by ECL to sensitize and fix the membrane. As a result, as shown in FIG. 9, amplification of 3 kb mCherry-Luciferase viral RNA was detected in proportion to the amount of pseudovirus product and expression of 180 kDa spike protein was detected.

**[Table 4]**

| | |
|---|---|
| Forward primer | GGGGGTGGGAAGCCCTCAAATATTGG (SEQ ID NO: 3) |
| Reverse primer | GGGTTGTGCCGCCTTTGCAGGTG (SEQ ID NO: 4) |

**[Table 5]**

| | |
|---|---|
| RT-PCR composition | Each forward primer 1 µL, vector 2.5 µL, dNTP 1 µL, 5X M-MLV buffer 4 µL, M-MLV Reverse Transcriptase 1 µL and distilled water 10.5 µL |
| RT-PCR conditions | Annealing: 65°C, 5 minutes |
| | Extension: 42°C, 1 hour |
| | Enzyme inactivation: 72°C, 15 minutes |

**[Table 6]**

| | |
|---|---|
| PCR composition | Each primer 1 µL, vector 1 µL, dNTP 5 µL, 10X KOD buffer 5 µL, MgSO4 3 µL, Phusion polymerase 1 µL and distilled water 31 µL |
| PCR conditions | Initial denaturation: 94°C, 2 minutes 35 cycles repetition: denaturation (98°C, 10 seconds) → extension (68°C, 3 minutes) |

### [Example 8: Antiviral activity of polymers and polymer nanodiscs against various groups of viruses]

### 1) Verification of antiviral efficacy against various strains of influenza virus

Comparison was performed using an MN assay in the same manner as in Example 4 in order to determine whether or not the polymer and polymer nanodisc exhibit antiviral efficacy against mutant influenza virus other than influenza virus (A/Puerto Rico/8/34). The result was serially diluted by 1/3 from the highest concentration of 30 µM based on the concentration of nanodisc phospholipids to the lowest concentration of 0.014 µM.

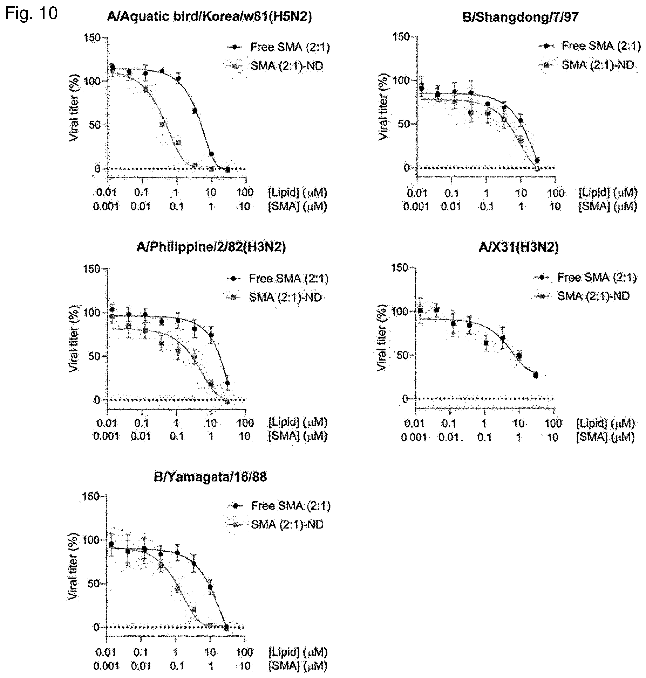

The result of quantitative analysis of the NA (neuraminidase) activity of virus showed that the nanodisc form exhibited 10-time lower IC₅₀ against influenza viruses (A/Philippine/2/82), (B/Yamagata/16/88), and (A/Aquatic bird/Korea/w81) compared to the polymer form, which indicates that the antiviral efficacy was increased (FIG. 10).

### 2) Verification of antiviral efficacy against VSV (vesicular stomatitis virus)-pseudovirus

When the 293T cell line was infected with VSV-pseudovirus and then treated with the nanodisc as an antiviral agent, the antiviral efficacy was determined by measuring the decrease in fluorescence and luminescence values due to inhibition of viral infection. Specifically, 500 µL of the 293T cell line was seeded at 3 X 105 cell/ml into each well of a 24-well cell culture plate and incubated in a 37°C and 5% CO2 incubator for 16 hours. Then, the medium was removed from the cells, 250 µL of PBS was seeded into each well, the medium was removed and then washing was performed. This process was repeated twice. 50 µL/well (1x105 RLU, relative light unit) of VSV-pseudovirus was prepared and mixed with various drugs (SMA-ND, heparin, lipo-G) at 2-fold dilution starting from 20 µM, followed by incubation at 37°C, and reaction in a 5% CO2 incubator for 1 hour. Then, each 293T cell well was treated with the result and transfected in an incubator at 37°C, 5% CO2 for 8 hours, the medium was replaced with fresh one, and the cells were incubated for an additional 48 hours. After culture, the medium was removed from the cells, and 250 µL of PBS was seeded into each well, removed, washed, and treated with 50 µL of 1X CCLR (cell culture lysis reagent, Promega, USA) per well. Then, each well of a 96-well white plate was treated with 20 µL of a cell lysate and 80 µL of luciferase assay buffer (Promega, USA), and the luminescence was measured using a spectrometer. 30 µL of cell lysate was added to each well of a 96-well black plate and fluorescence was measured at an excitation wavelength of 487 nm and an emission wavelength of 520 nm. At this time, the product obtained by culturing only the VSV-pseudovirus and the 293T cell line without an antiviral drug was used as a negative control group, the product obtained by culturing only the 293T cell line without viral infection was used as a positive control group, and conventional heparin sulfate (Galen, USA), which is known to have antiviral activity against VSV, was used as a control group. The decreases in fluorescence and luminescence caused by the nanodisc of these groups were measured.

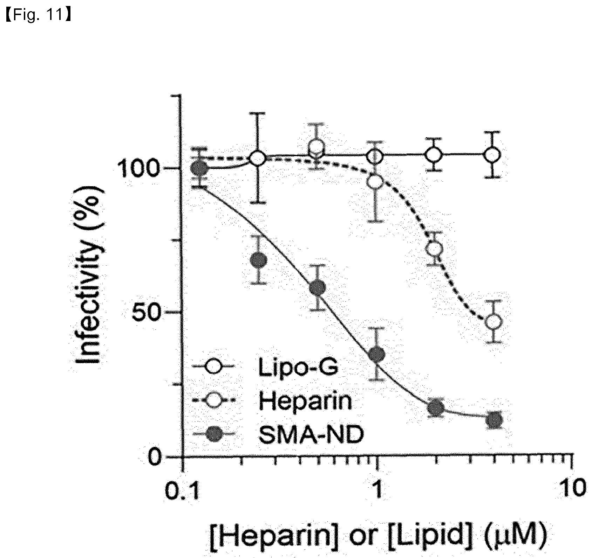

The result of the pseudovirus inhibition experiment showed that SMA-ND inhibited VSV-pseudovirus infection in proportion to the concentration and exhibited excellent inhibitory activity against viral infection compared to heparin and lipo-G (FIG. 11).

### 3) Verification of antiviral efficacy against coronavirus-pseudovirus

A coronavirus-pseudovirus inhibition experiment was performed using the coronavirus-pseudovirus and the nanodisc produced in Example in the same manner as in the verification of the antiviral activity of the VSV-pseudovirus, and the results were shown in a graph. Here, Camostat mesylate (Sigma, USA) was used as a control group, and the drug concentration and pseudovirus dose were the same as those in the verification of the antiviral activity of VSV-pseudovirus.

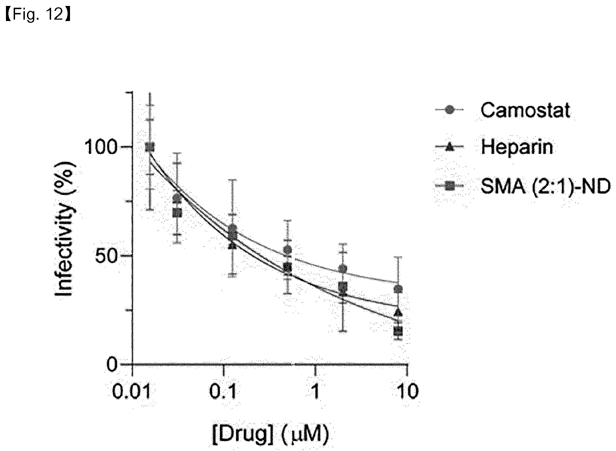

The result showed that SMA-ND exhibited inhibitory activity against coronavirus-pseudovirus infection in proportion to the concentration, and compared to conventional heparin and camostat, SMA-ND exhibited inhibitory activity of 22% against viral infection at the lowest concentration of 0.5 µM and 81% at the highest concentration of 2 µM (FIG. 12).

### [Example 9: Verification of antiviral effects of polymer nanodiscs through animal experiments]

Efficacy verification experiments were conducted on polymer nanodiscs that exhibited extremely high antiviral activity at the cellular level in experimental animal models ((A) and (B) of FIGS. 13). 7-week-old female BALB/c mice were used in the experiment and were challenged with PR8 virus of 4 MLD50 via intranasal (IN) route. 1 hour after the challenge, the same drug was administered intranasally via the IN route, once per day for two days, and the concentration of the administered SMA (2:1)-ND (DMPC) was 0.35 mg/kg. After the challenge, weight changes and survival rates were observed and recorded for 12 days. As a result, the experimental group administered polymer (SMA) exhibited a survival rate of 0%, while the experimental group administered the polymer nanodisc exhibited a survival rate of 60% ((A) of FIG. 13).

Meanwhile, the result of weight change analysis showed that the difference between the groups is even more noticeable. Unlike other experimental groups, which exhibited rapid weight loss about 2 days after virus inoculation, the experimental group administered polymer nanodiscs exhibited a relatively rapid recovery (see (B) of FIG. 13).

Overall, according to the embodiments of the present invention, the polymer nanodisc, especially SMA-ND, i) does not have cytotoxicity, ii) has excellent stability, and iii) has antiviral activity against various viruses such as influenza virus, coronavirus, and vesicular stomatitis virus (VSV). In addition, the polymer nanodisc of the present invention is considered to exhibit superior antiviral activity to the nanodisc produced using the membrane protein (MSP, membrane scaffold protein).

## Claims

1. A polymer nanodisc for use in preventing or treating a viral infection having a configuration in which an amphipathic polymer is bound to a lipid bilayer including hydrophobic moieties of amphiphilic lipids contacting each other.

2. The polymer nanodisc for use in preventing or treating a viral infection according to claim 1, wherein the polymer nanodisc has a configuration in which the hydrophobic moieties of amphiphilic lipids contacting each other are arranged as a bilayer including upper and lower layers to form a disc-shaped lipid bilayer, and parts of the hydrophobic moieties of the amphiphilic lipids exposed from side surfaces of the amphiphilic lipids are surrounded by an amphipathic polymer through a hydrophobic bond.

3. The polymer nanodisc for use in preventing or treating a viral infection according to claim 1, wherein the amphiphilic lipid comprises at least one phospholipid selected from the group consisting of phosphatidylcholine, phosphatidylglycerol, phosphatidylethanolamine, and phosphatidylserine.

4. The polymer nanodisc for use in preventing or treating a viral infection according to claim 1, wherein the amphiphilic lipid comprises at least one selected from the group consisting of DOPC (1,2-dioleoyl-sn-glycero-3-phosphocholine, DLPC (1,2-dilauroyl-sn-glycero-3-phosphocholine), DMPC (1,2-dimyristoyl-sn-glycero-3-phosphocholine), DPPC (1,2-dipalmitoyl-sn-glycero-3-phosphocholine), POPC (1-palmitoyl-2-oleoyl-sn-glycero-3-phosphocholine), C13PC, DDPC (1,2-didecanoyl-sn-glycero-3-phosphocholine), DSPC (1,2-distearoyl-sn-glycero-3-phosphocholine), DEPC (1,2-dierucoyl-sn-glycero-3-phosphocholine), DLOPC (1,2-dilinoleoyl-sn-glycero-3-phosphocholine), EPC (egg phosphatidylcholine), MSPC (1-myristoyl-2-stearoyl-sn-glycero-3-phosphocholine), PMPC (1-palmitoyl-2-myristoyl-sn-glycero-3-phosphocholine), PSPC (1-palmitoyl-2-stearoyl-sn-glycero-3-phosphocholine), SMPC (1-stearoyl-2-myristoyl-sn-glycero-3-phosphocholine), SPPC (1-stearoyl-2-palmitoyl-sn-glycero-3-phosphocholine) and combinations thereof.

5. The polymer nanodisc for use in preventing or treating a viral infection according to claim 1, wherein the amphiphilic polymer is present in an amount of 0.1 to 20% based on a total weight of the polymer nanodisc.

6. The polymer nanodisc for use in preventing or treating a viral infection according to claim 1, wherein the polymer nanodisc perforates a lipid bilayer envelope of a virus.

7. The polymer nanodisc for use in preventing or treating a viral infection according to claim 6, wherein the virus comprises at least one selected from the group consisting of the families *Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae* and *Herpesviridae.*

8. The polymer nanodisc for use in preventing or treating a viral infection according to claim 6, wherein the virus comprises at least one selected from the group consisting of influenza virus, coronavirus, and vesicular stomatitis virus.

9. A pharmaceutical composition for use in preventing or treating a viral infection comprising the polymer nanodisc for use in preventing or treating a viral infection according to any one of claims 1 to 8.

10. A method for screening an optimized polymer nanodisc structure composition comprising:
reacting a polymer nanodisc having a configuration in which an amphipathic polymer is bound to a lipid bilayer including hydrophobic moieties of amphiphilic lipids contacting each other with a target virus (a); and
determining whether or not the polymer nanodisc exhibits antiviral activity against the target virus (b).

11. The method according to claim 10, wherein the polymer nanodisc has a configuration in which the hydrophobic moieties of amphiphilic lipids contacting each other are arranged as a bilayer including upper and lower layers to form a disc-shaped lipid bilayer, and parts of the hydrophobic moieties of the amphiphilic lipids exposed from side surfaces of the amphiphilic lipids are surrounded by an amphipathic polymer through a hydrophobic bond.

12. The method according to claim 10, wherein the amphiphilic lipid comprises at least one phospholipid selected from the group consisting of phosphatidylcholine, phosphatidylglycerol, phosphatidylethanolamine, and phosphatidylserine.

13. The method according to claim 10, wherein the amphiphilic lipid comprises at least one selected from the group consisting of DOPC (1,2-dioleoyl-sn-glycero-3-phosphocholine, DLPC (1,2-dilauroyl-sn-glycero-3-phosphocholine), DMPC (1,2-dimyristoyl-sn-glycero-3-phosphocholine), DPPC (1,2-dipalmitoyl-sn-glycero-3-phosphocholine), POPC (1-palmitoyl-2-oleoyl-sn-glycero-3-phosphocholine), C13PC, DDPC (1,2-didecanoyl-sn-glycero-3-phosphocholine), DSPC (1,2-distearoyl-sn-glycero-3-phosphocholine), DEPC (1,2-dierucoyl-sn-glycero-3-phosphocholine), DLOPC (1,2-dilinoleoyl-sn- glycero-3-phosphocholine), EPC (egg phosphatidylcholine), MSPC (1-myristoyl-2-stearoyl-sn-glycero-3-phosphocholine), PMPC (1-palmitoyl-2-myristoyl- - glycero-3-phosphocholine), PSPC (1-palmitoyl-2-stearoyl glycero-3-phosphocholine), SMPC (1-stearoyl-2-myristoyl-sn-glycero-3-phosphocholine), SPPC (1-stearoyl-2-palmitoyl-sn-glycero-3-phosphocholine) and combinations thereof.

14. The method according to claim 10, wherein the amphiphilic polymer is present in an amount of 0.1 to 20% based on a total weight of the polymer nanodisc.

## Patentansprüche

1. Polymer-Nanodisc zur Verwendung bei der Vorbeugung oder Behandlung einer Virusinfektion, mit einer Konfiguration, in der ein amphipathisches Polymer an eine Lipid-Doppelschicht gebunden ist, wobei die Lipid-Doppelschicht hydrophobe Gruppen amphiphiler Lipide umfasst, die einander kontaktieren.

2. Polymer-Nanodisc zur Verwendung bei der Vorbeugung oder Behandlung einer Virusinfektion nach Anspruch 1, wobei der Polymer-Nanodisc eine Konfiguration aufweist, in der die einander kontaktierenden hydrophoben Gruppen amphiphiler Lipide als Doppelschicht angeordnet sind, die obere und untere Schichten umfasst, um eine scheibenförmige Lipid-Doppelschicht zu bilden, und Teile der hydrophoben Gruppen der amphiphilen Lipide, die von Seitenflächen der amphiphilen Lipide exponiert sind, durch eine hydrophobe Bindung von einem amphipathischen Polymer umgeben sind.

3. Polymer-Nanodisc zur Verwendung bei der Vorbeugung oder Behandlung einer Virusinfektion nach Anspruch 1, wobei das amphiphile Lipid zumindest ein Phospholipid umfasst, ausgewählt aus der Gruppe bestehend aus Phosphatidylcholin, Phosphatidylglycerol, Phosphatidylethanolamin und Phosphatidylserin.

4. Polymer-Nanodisc zur Verwendung bei der Vorbeugung oder Behandlung einer Virusinfektion nach Anspruch 1, wobei das amphiphile Lipid zumindest eines umfasst, ausgewählt aus der Gruppe bestehend aus DOPC (1,2-dioleoyl-sn-glycero-3-phosphocholine, DLPC (1,2-dilauroyl-sn-glycero-3-phosphocholine), DMPC (1,2-dimyristoyl-sn-glycero-3-phosphocholine), DPPC (1,2-dipalmitoyl-sn-glycero-3-phosphocholine), POPC (1-palmitoyl-2-oleoyl-sn-glycero-3-phosphocholine), C13PC, DDPC (1,2-didecanoyl-sn-glycero-3-phosphocholine), DSPC (1,2-distearoyl-sn-glycero-3-phosphocholine), DEPC (1,2-dierucoyl-sn-glycero-3-phosphocholine), DLOPC (1,2-dilinoleoyl-sn-glycero-3-phosphocholine), EPC (Eiphosphatidylcholin), MSPC (1-myristoyl-2-stearoyl-sn-glycero-3-phosphocholine), PMPC (1-palmitoyl-2-myristoyl-sn-glycero-3-phosphocholine), PSPC (1-palmitoyl-2-stearoyl-sn-glycero-3-phosphocholine), SMPC (1-stearoyl-2-myristoyl-sn-glycero-3-phosphocholine), SPPC (1-stearoyl-2-palmitoyl-sn-glycero-3-phosphocholine) und Kombinationen davon.

5. Polymer-Nanodisc zur Verwendung bei der Vorbeugung oder Behandlung einer Virusinfektion nach Anspruch 1, wobei das amphiphile Polymer in einer Menge von 0,1 bis 20 % bezogen auf ein Gesamtgewicht des Polymer-Nanodisc vorliegt.

6. Polymer-Nanodisc zur Verwendung bei der Vorbeugung oder Behandlung einer Virusinfektion nach Anspruch 1, wobei der Polymer-Nanodisc eine Lipid-Doppelschicht-Hülle eines Virus perforiert.

7. Polymer-Nanodisc zur Verwendung bei der Vorbeugung oder Behandlung einer Virusinfektion nach Anspruch 6, wobei das Virus zumindest eines umfasst, ausgewählt aus der Gruppe bestehend aus den Familien Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae und Herpesviridae.

8. Polymer-Nanodisc zur Verwendung bei der Vorbeugung oder Behandlung einer Virusinfektion nach Anspruch 6, wobei das Virus zumindest eines umfasst, ausgewählt aus der Gruppe bestehend aus Influenzavirus, Coronavirus und vesikulärem Stomatitisvirus.

9. Pharmazeutische Zusammensetzung zur Verwendung bei der Vorbeugung oder Behandlung einer Virusinfektion, umfassend den Polymer-Nanodisc zur Verwendung bei der Vorbeugung oder Behandlung einer Virusinfektion nach einem der Ansprüche 1 bis 8.

10. Verfahren zum Screening einer optimierten Polymer-Nanodisc-Strukturzusammensetzung, umfassend:
Reagierenlassen eines Polymer-Nanodisc, der eine Konfiguration aufweist, in der ein amphipathisches Polymer an eine Lipid-Doppelschicht gebunden ist, die hydrophobe Gruppen amphiphiler Lipide umfasst, die einander kontaktieren, mit einem Zielvirus (a); und
Bestimmen, ob der Polymer-Nanodisc eine antivirale Aktivität gegen das Zielvirus zeigt oder nicht (b).

11. Verfahren nach Anspruch 10, wobei der Polymer-Nanodisc eine Konfiguration aufweist, in der die einander kontaktierenden hydrophoben Gruppen amphiphiler Lipide als Doppelschicht angeordnet sind, die obere und untere Schichten umfasst, um eine scheibenförmige Lipid-Doppelschicht zu bilden, und Teile der hydrophoben Gruppen der amphiphilen Lipide, die von Seitenflächen der amphiphilen Lipide exponiert sind, durch eine hydrophobe Bindung von einem amphipathischen Polymer umgeben sind.

12. Verfahren nach Anspruch 10, wobei das amphiphile Lipid zumindest ein Phospholipid umfasst, ausgewählt aus der Gruppe bestehend aus Phosphatidylcholin, Phosphatidylglycerol, Phosphatidylethanolamin und Phosphatidylserin.

13. Verfahren nach Anspruch 10, wobei das amphiphile Lipid zumindest eines umfasst, ausgewählt aus der Gruppe bestehend aus DOPC (1,2-dioleoyl-sn-glycero-3-phosphocholine, DLPC (1,2-dilauroyl-sn-glycero-3-phosphocholine), DMPC (1,2-dimyristoyl-sn-glycero-3-phosphocholine), DPPC (1,2-dipalmitoyl-sn-glycero-3-phosphocholine), POPC (1-palmitoyl-2-oleoyl-sn-glycero-3-phosphocholine), C13PC, DDPC (1,2-didecanoyl-sn-glycero-3-phosphocholine), DSPC (1,2-distearoyl-sn-glycero-3-phosphocholine), DEPC (1,2-dierucoyl-sn-glycero-3-phosphocholine), DLOPC (1,2-dilinoleoyl-sn- glycero-3-phosphocholine), EPC (Eiphosphatidylcholin), MSPC (1-myristoyl-2-stearoyl-sn-glycero-3-phosphocholine), PMPC (1-palmitoyl-2-myristoyl- - glycero-3-phosphocholine), PSPC (1-palmitoyl-2-stearoyl glycero-3-phosphocholine), SMPC (1-stearoyl-2-myristoyl-sn-glycero-3-phosphocholine), SPPC (1-stearoyl-2-palmitoyl-sn-glycero-3-phosphocholine) und Kombinationen davon.

14. Verfahren nach Anspruch 10, wobei das amphiphile Polymer in einer Menge von 0,1 bis 20 % bezogen auf ein Gesamtgewicht des Polymer-Nanodisc vorliegt.

## Revendications

1. Nanodisque polymère destiné à être utilisé dans la prévention ou le traitement d'une infection virale, ayant une configuration dans laquelle un polymère amphipathique est lié à une bicouche lipidique, la bicouche lipidique comprenant des groupes hydrophobes de lipides amphiphiles qui se touchent mutuellement.

2. Nanodisque polymère destiné à être utilisé dans la prévention ou le traitement d'une infection virale selon la revendication 1, dans lequel le nanodisque polymère a une configuration dans laquelle les groupes hydrophobes de lipides amphiphiles se touchant mutuellement sont disposés en bicouche comprenant des couches supérieure et inférieure de manière à former une bicouche lipidique en forme de disque, et des parties des groupes hydrophobes des lipides amphiphiles exposées depuis des faces latérales des lipides amphiphiles sont entourées par un polymère amphipathique au moyen d'une liaison hydrophobe.

3. Nanodisque polymère destiné à être utilisé dans la prévention ou le traitement d'une infection virale selon la revendication 1, dans lequel le lipide amphiphile comprend au moins un phospholipide choisi dans le groupe consistant en la phosphatidylcholine, la phosphatidylglycérol, la phosphatidyléthanolamine et la phosphatidylsérine.

4. Nanodisque polymère destiné à être utilisé dans la prévention ou le traitement d'une infection virale selon la revendication 1, dans lequel le lipide amphiphile comprend au moins un élément choisi dans le groupe consistant en DOPC (1,2-dioleoyl-sn-glycero-3-phosphocholine), DLPC (1,2-dilauroyl-sn-glycero-3-phosphocholine), DMPC (1,2-dimyristoyl-sn-glycero-3-phosphocholine), DPPC (1,2-dipalmitoyl-sn-glycero-3-phosphocholine), POPC (1-palmitoyl-2-oleoyl-sn-glycero-3-phosphocholine), C13PC, DDPC (1,2-didecanoyl-sn-glycero-3-phosphocholine), DSPC (1,2-distearoyl-sn-glycero-3-phosphocholine), DEPC (1,2-dierucoyl-sn-glycero-3-phosphocholine), DLOPC (1,2-dilinoleoyl-sn-glycero-3-phosphocholine), EPC (phosphatidylcholine d'œuf), MSPC (1-myristoyl-2-stearoyl-sn-glycero-3-phosphocholine), PMPC (1-palmitoyl-2-myristoyl-sn-glycero-3-phosphocholine), PSPC (1-palmitoyl-2-stearoyl-sn-glycero-3-phosphocholine), SMPC (1-stearoyl-2-myristoyl-sn-glycero-3-phosphocholine), SPPC (1-stearoyl-2-palmitoyl-sn-glycero-3-phosphocholine) et des combinaisons de ceux-ci.

5. Nanodisque polymère destiné à être utilisé dans la prévention ou le traitement d'une infection virale selon la revendication 1, dans lequel le polymère amphiphile est présent en une quantité de 0,1 à 20 % sur la base d'un poids total du nanodisque polymère.

6. Nanodisque polymère destiné à être utilisé dans la prévention ou le traitement d'une infection virale selon la revendication 1, dans lequel le nanodisque polymère perfore une enveloppe de bicouche lipidique d'un virus.

7. Nanodisque polymère destiné à être utilisé dans la prévention ou le traitement d'une infection virale selon la revendication 6, dans lequel le virus comprend au moins un élément choisi dans le groupe consistant en les familles Bunyaviridae, Coronaviridae, Filoviridae, Flaviviridae, Hepadnaviridae, Herpesviridae, Orthomyxoviridae, Poxviridae, Rhabdoviridae, Retroviridae, Togaviridae et Herpesviridae.

8. Nanodisque polymère destiné à être utilisé dans la prévention ou le traitement d'une infection virale selon la revendication 6, dans lequel le virus comprend au moins un élément choisi dans le groupe consistant en le virus de la grippe, le coronavirus et le virus de la stomatite vésiculaire.

9. Composition pharmaceutique destinée à être utilisée dans la prévention ou le traitement d'une infection virale, comprenant le nanodisque polymère destiné à être utilisé dans la prévention ou le traitement d'une infection virale selon l'une des revendications 1 à 8.

10. Procédé de criblage d'une composition de structure optimisée de nanodisque polymère, comprenant :
faire réagir un nanodisque polymère, ayant une configuration dans laquelle un polymère amphipathique est lié à une bicouche lipidique comprenant des groupes hydrophobes de lipides amphiphiles qui se touchent mutuellement, avec un virus cible (a) ; et
déterminer si le nanodisque polymère présente ou non une activité antivirale contre le virus cible (b).

11. Procédé selon la revendication 10, dans lequel le nanodisque polymère a une configuration dans laquelle les groupes hydrophobes de lipides amphiphiles se touchant mutuellement sont disposés en bicouche comprenant des couches supérieure et inférieure de manière à former une bicouche lipidique en forme de disque, et des parties des groupes hydrophobes des lipides amphiphiles exposées depuis des faces latérales des lipides amphiphiles sont entourées par un polymère amphipathique au moyen d'une liaison hydrophobe.

12. Procédé selon la revendication 10, dans lequel le lipide amphiphile comprend au moins un phospholipide choisi dans le groupe consistant en la phosphatidylcholine, la phosphatidylglycérol, la phosphatidyléthanolamine et la phosphatidylsérine.

13. Procédé selon la revendication 10, dans lequel le lipide amphiphile comprend au moins un élément choisi dans le groupe consistant en DOPC (1,2-dioleoyl-sn-glycero-3-phosphocholine), DLPC (1,2-dilauroyl-sn-glycero-3-phosphocholine), DMPC (1,2-dimyristoyl-sn-glycero-3-phosphocholine), DPPC (1,2-dipalmitoyl-sn-glycero-3-phosphocholine), POPC (1-palmitoyl-2-oleoyl-sn-glycero-3-phosphocholine), C13PC, DDPC (1,2-didecanoyl-sn-glycero-3-phosphocholine), DSPC (1,2-distearoyl-sn-glycero-3-phosphocholine), DEPC (1,2-dierucoyl-sn-glycero-3-phosphocholine), DLOPC (1,2-dilinoleoyl-sn-glycero-3-phosphocholine), EPC (phosphatidylcholine d'œuf), MSPC (1-myristoyl-2-stearoyl-sn-glycero-3-phosphocholine), PMPC (1-palmitoyl-2-myristoyl-sn-glycero-3-phosphocholine), PSPC (1-palmitoyl-2-stearoyl-sn-glycero-3-phosphocholine), SMPC (1-stearoyl-2-myristoyl-sn-glycero-3-phosphocholine), SPPC (1-stearoyl-2-palmitoyl-sn-glycero-3-phosphocholine) et des combinaisons de ceux-ci.

14. Procédé selon la revendication 10, dans lequel le polymère amphiphile est présent en une quantité de 0,1 à 20 % sur la base d'un poids total du nanodisque polymère.
